# EUROPEAN PATENT APPLICATION

(11) **EP 4 803 430 A1**
(43) Date of publication of application: **09.09.2026**
(21) Application number: 26160673.5
(22) Date of filing: 25.02.2026
(51) Int. Cl.: B65B 55/10, B65B 3/04, A61L 2/10, A61L 2/24, B31B 50/74, B65B 55/02, B65B 55/08, B67C 7/00

(54) **STERILIZATION OF PACKAGING MATERIAL WITHIN A PACKAGING MACHINE FOR FILLING PACKAGES WITH FOOD PRODUCTS**

(30) Priority: 06.03.2025 EP 25162016
(71) Applicant: Tetra Laval Holdings & Finance S.A., 1009 Pully (CH)
(72) Inventor: Lindblad, Jenny, 221 86 Lund (SE); Lindblad, Ulf, 221 86 Lund (SE); Floderus, Anders, 221 86 Lund (SE)
(74) Representative: Tetra Pak Patent Attorneys

(57) **Abstract**

The present invention relates to a sterilization arrangement for sterilization of packaging material within a packaging machine configured to fill packages formed from the packaging material with a food product. The packaging material comprising specific areas that occur repetitively upon the packaging material being fed through the packaging machine. The sterilization arrangement comprises a UV-light irradiation station comprising a plurality of UV-LED units arranged along a feeding direction of the packaging material. The plurality of UV-LED units are configured to be individually controllable. The sterilization arrangement further comprises a control circuitry configured to individually adjust an UV-light irradiation intensity of the plurality of UV-LED units as a specific area among the specific areas that occur repetitively upon the packaging material being fed through the sterilization arrangement passes through the UV-light irradiation station. A packaging machine configured to fill packages with a food product, the packaging machine comprising the sterilization arrangement, is also presented.

## Description

### Technical field

The present invention relates to sterilization of packaging material within a packaging machine for filling packages with food products, typically liquid food products. Especially, sterilization of specific areas that occurs repetitively in the packaging material as the packaging material being feed through the packaging machine. The sterilization is achieved by means of UV-light irradiation. It is further to be noted that whenever sterilization is discussed herein, it also applies to disinfection, depending on the target for the irradiation using UV-light.

### Background of the invention

Within the food industry today, the food package technology plays an important part. The food package has several important functions. Apart from branding of the product and presenting the customers with information, the food package also has an important role of ensuring food safety. The packaging materials used in the food package can be designed to provide strength and stability, so that the packages are not damaged during transportation. Furthermore, the packaging materials can form a protective environment for the food product so that it is protected from for example bacteria, germs, oxygen and sun light, thus prolonging shelf life. However, the packaging material is not the only thing that is important in the package. The package need to be subjected to sterilization before filling the package with food. This in order to increase shelf life of the food in the package and to ensure food safety.

Performing sterilization of packaging material may be challenging and a variety of sterilization methods, including thermal methods, such as steam sterilization and hot air sterilization, chemical methods, such as hydrogen peroxide (H₂O₂) sterilization and peracetic acid sterilization, and irradiation methods, such as gamma irradiation, electron beam irradiation and ultra violet, UV, light irradiation, have been implemented in the industry. Current sterilization methods of packaging material leave room for improvements, especially when it comes to high speed packaging machines in which a time window for performing the sterilization is narrow.

### Summary of the invention

The herein disclosed technology seeks to at least partly mitigate, alleviate or eliminate one or more of the above-mentioned deficiencies and disadvantages in the prior art. In particular, it is an object to provide efficient sterilization of packaging material within a packaging machine. The inventors of the present inventive concept has realized a new and improved way of sterilizing packaging material, especially in connection with high speed filling in a packaging machine. By high speed filling is here meant filling of at least 4500 packages per hour per packaging line of the filling machine.

Various aspects and embodiments of the disclosed invention are defined below and in the accompanying independent and dependent claims.

According to a first aspect, a sterilization arrangement for sterilization of packaging material within a packaging machine configured to fill packages formed from the packaging material with a food product is presented. The packaging material comprising specific areas that occurs repetitively upon the packaging material being feed through the packaging machine. The sterilization arrangement comprises an UV-light irradiation station comprising a plurality of UV-LED units arranged along a feeding direction of the packaging material. The plurality of UV-LED units are configured to be individually controllable. The sterilization arrangement further comprises a control circuitry configured to individually adjust an UV-light irradiation intensity of the plurality of UV-LED units as a specific area among the specific areas that occurs repetitively upon the packaging material being feed through the sterilization arrangement passes through the UV-light irradiation station.

As will be discussed in more detail below, the packaging material may be in the form of a web of packaging material. Alternatively, the packaging material may be in the form blanks or unfilled bottes.

The UV-light irradiation station according to the present invention is hence configured to subject specific areas that occurs repetitively in the packaging material, upon the packaging material being feed through the UV-light irradiation station, for UV-light. In case the packaging material being in form of a web of packaging material the specific areas occurs repetitively in the web. In case the packaging material being in form of a plurality of blanks, specific areas occur one or more times for each blank being feed through the UV-light irradiation station. In case the packaging material being in form of bottles, specific areas occur one or more times for each bottle being feed through the UV-light irradiation station. Hence, the UV-light irradiation station is configured to subject the specific areas of the packaging material for UV-light irradiation as the packaging material is being feed through the sterilization arrangement. By configuring the UV-light irradiation station to individually adjust the UV-light irradiation intensity of the plurality of UV-LED units as a specific area being feed through the sterilization arrangement allow for specific treatment of the specific area. In other words, a specific UV-LED unit may be controlled to emit a relatively higher intensity of UV-light upon a specific area is in an irradiation zone of the specific UV-LED unit and to be controlled to emit a relatively lower intensity of UV-light upon the specific areas are outside the irradiation zone of the specific UV-LED unit. Hence, the specific controlling of the plurality of UV-LED units presented herein allow for reduced need of cooling the UV-LED units. This since they do not need to be fully turned on all the time. Further, a lifetime of the UV-LED units may be increased. Also, this due to that they do not need to be fully turned on all the time. Moreover, UV-dosage to the specific areas may be better controlled so that no overdosing is made allowing more delicate materials in the packaging material to be used. With delicate materials is hereby meant materials that may be negatively affected by being exposed for a "too high" UV-dosage.

The specific areas may be a type of area selected from the group of areas consisting of: crease lines, embossed or debossed patterns, seal zones, reinforcement strips, opening device packaging material interfaces, and opening devices.

The UV-light irradiation station may be a stationary station.

The UV-light irradiation station may comprise a two dimensional matrix of UV-LED units. The matrix may comprise a first number of columns of UV-LED units arranged along the feeding direction of packaging material. The matrix may further comprise a second number of rows of UV-LED units arranged transverse the feeding direction of packaging material. The rows of UV-LED units may be configured to be individually controllable. Accordingly, one or more rows of UV-LED units may be controlled as a specific area passes through the UV-light irradiation station. Hence, the control circuitry may be configured to adjust an UV-light irradiation intensity of the rows of UV-LED units as a specific area passes through the UV-light irradiation station.

The UV-LED units in each row of UV-LED units may be configured to be individually controllable. The control circuitry may be configured to individually control which UV-LED units in the rows of UV-LED units to be adjusted based on one or both of a position and a size of the specific area along a direction transverse the feeding direction of the packaging material.

The sterilization arrangement may further comprise an additional UV-light irradiation station configured to subject the packaging material for UV-light irradiation along a full extension of the direction transverse the feeding direction of the packaging material.

Each UV-LED unit may comprise one or more individual UV-LEDs.

The UV-LED units are arranged to emit UV-light within the UVC spectral range.

According to a second aspect packaging machine configured to fill packages with a food product is presented. The packaging machine comprises a sterilization arrangement according to the first aspect, and a feed unit configured to feed the packaging material through the UV-light irradiation station of the sterilization arrangement.

The packaging material may be in the form of a web of packaging material. The feed unit may be configured to continuously feed the web of packaging material through the UV-light irradiation station of the sterilization arrangement.

The control circuitry of the sterilization arrangement may be configured to individually adjust an UV-light irradiation intensity of the UV-LED units arranged along the feeding direction of packaging material based on a speed at which the feed unit is feeding the packaging material through the UV-light irradiation station.

The above mentioned features of the first aspect, when applicable, apply to this second aspect as well. In order to avoid undue repetition, reference is made to the above.

According to a third aspect a method for, within a packaging machine configured to fill packages with a food product, sterilization of packaging material comprising specific areas that occurs repetitively as the packaging material is fed through the packaging machine is presented. The method comprises subjecting, at a UV-light irradiation station comprising a plurality of UV-LED units arranged along a feeding direction of packaging material, the specific areas for UV-light irradiation by individually adjusting an UV-light irradiation intensity of the plurality of UV-LED units as a specific area passes through the UV-light irradiation station.

The UV-light irradiation station may be a stationary station. The packaging material may be in the form of a web of packaging material. The method may further comprise continuously feeding the web of packaging material comprising the specific areas through the UV-light irradiation station.

Individually adjusting the UV-light irradiation intensity of the plurality of UV-LED units as a specific area passes through the UV-light irradiation station may be performed based on a feeding speed of the packaging material through the UV-light irradiation station.

The above mentioned features of the first and second aspects, when applicable, apply to this third aspect as well. In order to avoid undue repetition, reference is made to the above.

A further scope of applicability of the present disclosure will become apparent from the detailed description given below. However, it should be understood that the detailed description and specific examples, while indicating preferred variants of the present inventive concept, are given by way of illustration only, since various changes and modifications within the scope of the inventive concept will become apparent to those skilled in the art from this detailed description.

### Brief description of the drawings

The above and other aspects of the present inventive concept will now be described in more detail, with reference to appended drawings showing variants of the present inventive concept. The figures should not be considered limiting the invention to the specific variant; instead, they are used for explaining and understanding the inventive concept.

As illustrated in the figures, the sizes of layers and regions are exaggerated for illustrative purposes and, thus, are provided to illustrate the general structures of variants of the present inventive concept. Like reference numerals refer to like elements throughout.
Fig. 1 schematically illustrates a packaging machine configured to fill packages with a liquid food product, the packaging machine being equipped with a packaging sterilization arrangement.
Fig. 2 schematically illustrates UV-light irradiation station comprising a matrix of LED-units being configured to be controlled individually or as subsets of LED-units.
Fig. 3 is a block diagram of a method for sterilization of packaging material within a packaging machine configured to fill packages with a food product, the method is specifically directed towards sterilization of specific areas that occurs repetitively in the packaging material as the packaging material being feed through the packaging machine.

### Detailed description

The present inventive concept will now be described more fully hereinafter with reference to the accompanying drawings, in which currently preferred variants of the inventive concept are shown. This inventive concept may, however, be implemented in many different forms and should not be construed as limited to the variants set forth herein; rather, these variants are provided for thoroughness and completeness, and fully convey the scope of the present inventive concept to the skilled person.

It will also be appreciated that when the present disclosure is described in terms of a method, it may also be embodied in an apparatus or device comprising one or more processors, one or more memories coupled to the one or more processors, where computer code is loaded to implement the method. For example, the one or more memories may store one or more computer programs that perform the steps, services and functions disclosed herein when executed by the one or more processors.

It is also to be understood that the terminology used herein is for the purpose of describing particular embodiments only, and is not intended to be limiting. It should be noted that, as used in the specification and the appended claim, the articles "a", "an", "the", and "said" are intended to mean that there are one or more of the elements unless the context clearly dictates otherwise. Thus, for example, reference to "a unit" or "the unit" may refer to more than one unit in some contexts, and the like. Furthermore, the words "comprising", "including", "containing" do not exclude other elements or steps. It should be emphasized that the term "comprises/comprising" when used in this specification is taken to specify the presence of stated features, integers, steps, or components. It does not preclude the presence or addition of one or more other features, integers, steps, components, or groups thereof. The term "and/or" is to be interpreted as meaning "both" as well and each as an alternative. The term "obtaining" is herein to be interpreted broadly and encompasses receiving, retrieving, collecting, acquiring, and so forth.

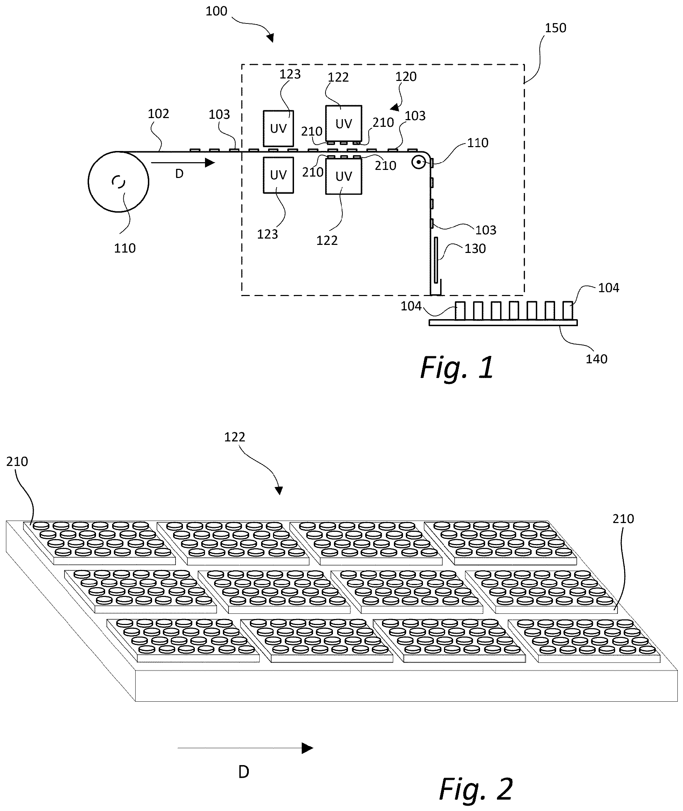

Fig. 1 generally illustrates, a packaging machine 100 configured to fill packages with a food product, according to some examples a liquid food product. The packaging machine 100 may be a roll-fed packaging machine used for producing packages. That is, the packaging machine 100 may be fed with a web 102 of packaging material to be formed into filled packages. The web 102 of packaging material may be flat. More specifically, the packaging machine 100 may be used for packaging food products in carton-based packages. Already in the 1940s this type of packaging machines was introduced by Tetra Pak^{®} and it is today a well-known approach for packaging milk and other food products in a safe and cost-efficient manner. The packaging material typically comprises a carton layer and at least one barrier layer designed to protect the product from external influences such as moisture, oxygen, light, and contaminants, which can degrade food quality and shelf life. The choice of barrier material depends on the specific requirements of the packaged food product and will not be elaborated upon in more detail in this text. The packaging material is often printed and prepared in packaging material production centers, also referred to as converting factories, and shipped to a site where the packaging machine 100 is placed, e.g. a dairy. For a roll-fed packaging machine 100 the packaging material is loaded onto a reel before being transported. After arriving at the site, the reel is placed in the packaging machine 100, and a web 102 of packaging material is fed in the packaging machine 100 by a feed unit 110. The feeding of the web 102 of packaging material is typically continuous.

Alternatively, to that the packaging machine 100 is a roll-fed packaging machine it may be a blank-fed packaging machine in which the packaging material is provided as blanks to the packaging machine 100. Such blanks are sometime referred to as preforms or carton blanks. Hence, the packaging material may be in the form of blanks. The blanks typically comprises a carton layer and at least one barrier layer designed to protect the product from external influences such as moisture, oxygen, light, and contaminants, which can degrade food quality and shelf life. The choice of barrier material depends on the specific requirements of the packaged food product and will not be elaborated upon in more detail in this text. At a production center for producing the blanks rolls of paperboard may be coated with one or more barrier layers, and possibly other layers. The coated material is printed with brand and product information, then cut into individual blanks and typically provided with folding creases. Once the blanks are produced, they are stacked and tightly packed in bundles to optimize storage and shipping. After arriving at the site of the packaging machine, the blanks are fed into the packaging machine. A blank-fed packaging machine is typically operated in an intermittent manner, however blank-fed packaging machine may alternatively be operated in a continuous manner. The flat blanks are folded into a ready-to-fill package shape, the edges are sealed using heat or adhesives. **In** aseptic systems, like in the system presented herein, sterilization is performed before filling. The formed and sterilized packages are filled with the intended liquid product, such as milk, juice, or soup. Thereafter the package is sealed.

Yet alternatively, the packing machine 100 may be bottling machine in which the packaging material is provided as empty bottles to the packaging machine 100. The bottles may be PET bottles.

Regardless of the type of packaging machine 100 it further comprises a packaging sterilization arrangement 120. The packaging sterilization arrangement 120 is configured to sterilize the packaging material before a package 104 is filled. It is further to be noted that whenever sterilization is discussed herein, it also applies to disinfection. In the in Fig.1 illustrated example, the packaging material is provided in the form of the web 102 of packaging material. However, in line with the discussion above, alternatively the packaging material may be provided as blanks to be formed and filled within the packaging machine or bottles to be filled within the packaging machine. Hence, the packaging sterilization arrangement 120 is configured to sterilize packaging material within the packaging machine 100 before a respective package is filled and possibly also before a respective package is formed. According to a specific example, typically in the case of a flat packaging material, the packaging sterilization arrangement 120 may be configured to sterilize both sides of the flat packaging material before the respective package is formed and filled.

The sterilization arrangement 120 will now be discussed in more detail. The sterilization arrangement 120 is configured to subject the packaging material to sterilization before filling a package formed from the packaging material. In many applications, the packaging material comprises specific areas 103 that occurs repetitively upon the packaging material being feed through the packaging machine 100. The specific areas 103 may be of various types. Some such types of specific areas 103 in the packaging material will be elaborated upon directly below. The specific areas 103 may be crease lines in the packaging material, the crease lines being used to aid in folding of the packaging material into packages. The specific areas 103 may be embossed or debossed patterns, i.e. raised or recessed features for branding, texturing, or functional purposes. The specific areas 103 may be seal zones, i.e. areas in the packaging material treated or coated to facilitate sealing during packaging. The specific areas 103 may be reinforcement strips, i.e. extra material layers or coatings to strengthen specific sections of the package. The specific areas 103 may be opening device packaging material interfaces, i.e. interfaces at which opening devices are to be applied to the packaging material. The specific areas 103 may be opening devices, e.g. pull tabs or spouts (with or without screw caps).

The sterilization arrangement 120 comprises an UV-light irradiation station 122. The UV-light irradiation station 122 is generally illustrated in more detail in connection with Fig. 2. The UV-light irradiation station 122 comprises a plurality of UV-LED units 210 arranged along a feeding direction D of the packaging material through the UV-light irradiation station 122. The plurality of UV-LED units 210 are configured to be individually controllable. By being individually controllable is in this context meant that an UV-light irradiation intensity out of the plurality of UV-LED units 210 is individually controllable for each UV-LED unit 210. According to a specific example, the UV-LED units 210 may be individually activated/deactivated, i.e. individually switched on / switched off. However, it is to be realized that the UV-LED units 210 may be individually regulated to emit a percentage of its nominal effect, such as 0%, 25%, 50%, 75% or 100%.

Each UV-LED unit 210 comprise one or more individual UV-LEDs. Typically, each UV-LED unit 210 comprises tens to hundreds of UV-LEDs. In the in Fig. 2 illustrated example, the UV-LED unit 210 comprises 24 UV-LEDs. The UV-LEDs are arranged to emit UV, light. Preferably, the UV-LEDs are arranged to emit UV-light having a peak wavelength in the UVC spectral region, i.e. within the wavelength range of 100 nm to 280 nm. According to one example, the UV-LEDs are arranged to emit UV-light having a peak wavelength in the range of 260-275 nm, more precisely in the range of 265-275 nm. Two examples of suitable UV-LEDs that can be used are OSRAM OSLON^{®} UV 6060, SU CZHEF1.VC and OSRAM OSLON^{®} UV 3535, SU CULEP1.VC. As understood by the skilled person, other UV-LEDs may of course be used. Further, as also understood by the skilled person, in the future UV-LEDs with higher power than available today will be developed. The UV-LEDs of an individual UV-LED unit 210 may all be arranged to emit UV-light with a same peak wavelength. Having, all UV-LEDs with the same peak wavelength will provide as high exposure as possible at that peak wavelength. Alternatively, the UV-LEDs of an individual UV-LED unit 210 may comprise two or more different types of UV-LEDs having different peak wavelengths. Having different peak wavelengths may be beneficial since different microorganism may have a different sensibility for different wavelengths. During operation of the UV-light irradiation station 122, the UV-LEDs are configured to emit UV-light towards a surface of the packaging material, especially towards a portion of the surface of the packaging material corresponding to the specific areas 103.

The sterilization arrangement 120 further comprises control circuitry configured to individually adjust an UV-light irradiation intensity of the plurality of UV-LED units 210 as a specific area 103, among the specific areas 103 that occurs repetitively upon the packaging material, being fed through the sterilization arrangement 120, passes through the UV-light irradiation station 122. Hence, the UV-light irradiation station 122 is configured to subject each specific area 103 that occurs repetitively in the packaging material upon the packaging material being fed through the UV-light irradiation station 122 for UV-light. In case the packaging material being in form of a web 102 of packaging material the specific areas 103 occur repetitively in the web 102. In case the packaging material being in form of a plurality of blanks, specific areas 103 occur one or more times for each blank being feed through the UV-light irradiation station 122. In case the packaging material being in form of a bottles, specific areas 103 occur one or more times for each bottle being feed through the UV-light irradiation station 122. Hence, the UV-light irradiation station 122 is configured to subject the specific areas 103 of the packaging material for UV-light irradiation as the packaging material is being fed through the sterilization arrangement 120.

Further, by configuring the UV-light irradiation station 122 to individually adjust the UV-light irradiation intensity of the plurality of UV-LED units 210 as a specific area 103 being feed through the sterilization arrangement 120 allow for specific treatment of the specific area 103. In other words, a specific UV-LED unit 210 may be controlled to emit a relatively higher intensity of UV-light upon a specific area 103 is in an irradiation zone of the specific UV-LED unit 210 and to be controlled to emit a relatively lower intensity of UV-light upon the specific areas 103 are outside the irradiation zone of the specific UV-LED unit 210. According to one use case, a specific UV-LED unit 210 may be controlled to be activated upon a specific area 103 is in the irradiation zone of the specific UV-LED unit 210 and to be deactivated upon the specific areas 103 are outside the irradiation zone of the specific UV-LED unit 210. Hence, the specific controlling of the plurality of UV-LED units 210 presented herein allow for reduced need of cooling the UV-LED units 210. This since they do not need to be fully turned on all the time. Further, a lifetime of the UV-LED units 210 may be increased. Also, this due to that they do not need to be fully turned on all the time. Moreover, UV-dosage to the specific areas 103 may be better controlled so that no overdosing is made allowing more delicate materials in the packaging material to be used. With delicate materials is hereby meant materials that may be negatively affected by being exposed for a "too high" UV-dosage.

With reference to Fig. 2 we will now look at the UV-light irradiation station 122 in more detail. The UV-light irradiation station 122 may comprise a two dimensional matrix of UV-LED units 210. The matrix comprises a first number of columns of UV-LED units 210 arranged along the feeding direction D of packaging material within the UV-light irradiation station 122. In the in Fig. 2 illustared example, the UV-light irradiation station 122 comprises three columns of UV-LED units 210 arranged along the feeding direction D of packaging material within the UV-light irradiation station 122. The matrix further comprises a second number of rows of UV-LED units 210 arranged transverse the feeding direction D of packaging material within the UV-light irradiation station 122. In the in Fig. 2 illustared example, the UV-light irradiation station 122 comprises four rows of UV-LED units 210 arranged transverse the feeding direction D of packaging material within the UV-light irradiation station 122. The rows of UV-LED units 210 may be configured to be individually controllable. For example, the control circuitry may be configured to individually adjust an UV-light irradiation intensity of each row of the number of rows of UV-LED units 210 as a specific area 103 within the packaging material passes through the UV-light irradiation station 122.

In other words, a specific row of UV-LED units 210 may be controlled to emit a relatively higher intensity of UV-light upon a specific area 103 is in an irradiation zone of the specific row of UV-LED units 210 and to be controlled to emit a relatively lower intensity of UV-light upon the specific areas 103 are outside the irradiation zone of the specific row of UV-LED units 210. According to one use case, a specific row of UV-LED units 210 may be controlled be activated upon a specific area 103 is in the irradiation zone of the specific row of UV-LED units 210 and to be deactivated upon the specific areas 103 are outside the irradiation zone of the specific row of UV-LED units 210.

Further, the UV-LED units 210 in each row of UV-LED units 210 may be configured to be individually controllable. The control circuitry may then be configured to individually control which UV-LED units 210 in each row of UV-LED units 210 to be adjusted based on one or both of a position and a size of the specific area 103 along a direction transverse the feeding direction D of the packaging material. This will allow for the sterilization arrangement 120 to be adopted for different sized/positioned specific areas 103 in the flow of packaging material through the UV-light irradiation station 122.

As being schematically illustrated in connection with Fig. 1, the sterilization arrangement 120 may comprise more than one UV-light irradiation stations 122. One of the UV-light irradiation stations 122 may be configured to irradiate the packaging material from above and another one of the UV-light irradiation stations 122 may be configured to irradiate the packaging material from below. The UV-light irradiation stations 122 configured to irradiate the packaging material from above and from below may be arranged to face each other. The set-up with UV-light irradiation stations 122 configured to irradiate the packaging material from above and from below is especially useful in a packaging machine configured to handle flat packaging material in the form of a web 102 of packaging material or in the form of blanks of packaging material. The above side of the web 102 of packaging material may be the side of the packaging material that will constitute an inside of the packages 104 being produced within the packaging machine 100. The below side of the packaging material may be the side of the packaging material that will constitute an outside of the packages 104 being produced within the packaging machine 100, or vice versa. Accordingly, sterilization arrangement 120 may be configured to irradiate both sides of the packaging material.

A distance between the UV-LEDs of a UV-LED unit 210 may be set depending on different factors. The UV-LEDs of a UV-LED unit 210 may be mounted on a Printed Circuit Board, PCB. In general, the distance between the UV-LEDs mounted on a PCB is selected to be as small as possible in order to maximize the irradiance (energy per area) delivered to the target. This smallest possible distance is determined by various tolerances. The LEDs are typically placed on the PCB by a pick-and-place machine, and then soldered to the PCB in an oven. During manufacturing tolerances in the size of the LEDs, the tolerance on the accuracy of the pick-and place machine, and the fact that the LEDs can move slightly during the soldering process in the oven as it slides on the melted solder are to be taken into consideration. Considering all of these tolerances, typically a minimum distance between LEDs is 0.3 mm.

Further, there are instances in which it is not desirable to place the LED units as close as possible on the PCB. This can be the case when lowering of the cooling requirements are needed. UV-LEDs generate heat during operation. When placed too close, the heat from each LED can accumulate, leading to higher temperatures. This can cause the LEDs to overheat, reducing their efficiency and lifespan. Increasing the spacing between the LEDs decreases this effect. Hence, spacing between individual UV-LEDs may be as much as some millimeters.

According to one example, the UV-LED unit 210 in the matrix are evenly distributed.

The UV-light irradiation station 122 is typically stationary within the packaging machine 100.

The sterilization arrangement 120 may further comprise an additional UV-light irradiation station 123. The additional UV-light irradiation station 123 is configured to subject the packaging material for UV-light irradiation along a full extension of the direction transverse the feeding direction D of the packaging material. In order to be able to subject the packaging material for UV-light irradiation along the full extension of the direction transverse the feeding direction D of the packaging material the additional UV-light irradiation station 123 comprises a plurality of UV-LEDs. The plurality of UV-LEDs are typically arranged along a line transverse the feeding direction D of the packaging material.

After having been sterilized, by means of the sterilization arrangement 120, the packaging material is filled with the food product at a filling station 130. The sterilized packaging material additionally may also be formed into packages 104 after having been sterilized. According to one specific embodiment, the packaging material is formed into a tube which is filled with the food product and thereafter being formed into packages 104. The filled packages 104 are then transported away from the packaging machine by a conveyor system 140.

The packaging machine may further comprise a sealing station configured to seal packages filled with the liquid food product. The packaging machine may be configured to fill at least 4500 packages per hour per packaging line. Hence, the packaging machine may be configured to high-speed filing.

Hence, the packaging machine 100 is configured to fill packages 104 with a food product. The packaging machine 100 comprises a sterilization arrangement 120 as discussed above and a feed unit 110 configured to feed the packaging material through the UV-light irradiation station 122 of the sterilization arrangement 120. The UV-light irradiation station 122 is configured to subject the packaging material for UV-light irradiation. Especially, a control circuitry of the sterilization arrangement 120 may individually control the plurality of UV-LED units 210 arranged along the feeding direction D of the packaging material such that an UV-light irradiation intensity of the plurality of UV-LED units 210 is adjusted as a specific area 103 of the packaging material being fed through the sterilization arrangement 120 passes through the UV-light irradiation station 122.

As generally illustrated in connection with Fig. 1, the packaging material may be in the form of a web 102 of packaging material. The feed unit 110 may be configured to continuously feed the web 102 of packaging material through the UV-light irradiation station 122 of the sterilization arrangement 120.

The control circuitry of the sterilization arrangement 120 may be configured to individually adjust an UV-light irradiation intensity of the UV-LED units 210 arranged along the feeding direction D of packaging material based on a speed at which the feed unit 110 is feeding the packaging material through the UV-light irradiation station 122. The individual controlling of the UV-LED units 210 may also be based on a position and/or size of the specific areas 103 that occurs repetitively as the packaging material is fed through the UV-light irradiation station 122.

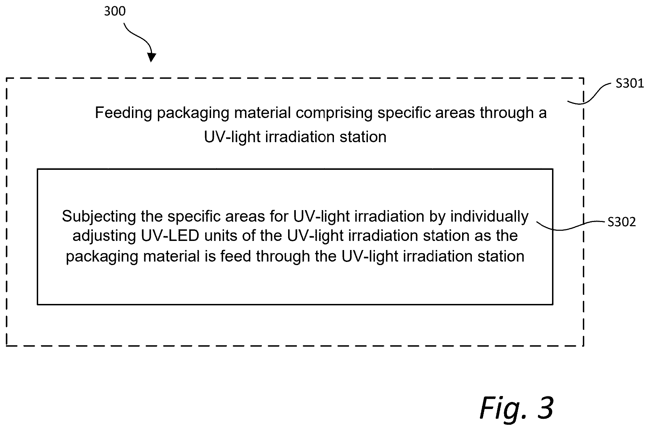

In connection with Fig. 3, a method 300 for sterilization of packaging material within a packaging machine configured to fill packages with a food product will be discussed. As discussed above, the packaging material comprises specific areas 103 that occur repetitively as the packaging material is fed through the packaging machine 100. Examples of different types of specific areas 103 are discussed above and reference is made to this discussion in order to avoid undue repetition. Below, the different steps of the method 300 will be described in more detail. The steps of the method 300 are performed within the packaging machine, e.g. the packaging machine 100 discussed in connection with Fig. 1. Some of the steps, or even all steps, of the method 300 may be executed by a control unit of the packaging machine.

The method 300 comprises subjecting S302, at the UV-light irradiation station 122 the specific areas 103 for UV-light irradiation by individually adjusting an UV-light irradiation intensity of the plurality of UV-LED units 210 as a specific area 103 passes through the UV-light irradiation station 122. Individually adjusting the UV-light irradiation intensity of the plurality of UV-LED units 210 as a specific area 103 passes through the UV-light irradiation station 122 may be performed based on a feeding speed of the packaging material through the UV-light irradiation station 122.

As discussed above, the UV-light irradiation station 122 may be a stationary station. Further, as also discussed above, the packaging material may be in the form of a web 102 of packaging material. The method 300 may further comprise continuously feeding S201 the web 102 of packaging material comprising the specific areas 103 through the UV-light irradiation station 122.

The person skilled in the art realizes that the present invention by no means is limited to what is explicitly described above. On the contrary, many modifications and variations are possible within the scope of the appended claims.

For example, in case the packaging material being in the form of blanks, the UV-light irradiation at the UV-light irradiation station 122 may be made on the blanks being in a flat state. However, the UV-light irradiation at the UV-light irradiation station 122 may be made on the blanks after the blanks have been formed into empty packages ready to be filled with the food product.

Further, the packaging machine 100 may comprise a clean air system 150. The clean air system 150 being arranged to enclosed the sterilization arrangement 120 and at least the portion of the feed unit 110 set to feed the packaging material through the UV-light irradiation station 122. The clean air system 150 may further enclose the filling station 130. As readily understood by the skilled person, alternative to a clean-air system 150 other means of creating an environment protecting the packaging material from recontamination between UV-light irradiation and filling/closing of the package 104 may be used.

Additionally, variations can be understood and effected by the skilled person in practicing the claimed invention, from a study of the drawings, the disclosure, and the appended claims.

## Claims

1. A sterilization arrangement (120) for sterilization of packaging material within a packaging machine (100) configured to fill packages (104) formed from the packaging material with a food product, the packaging material comprising specific areas (103) that occurs repetitively upon the packaging material being fed through the packaging machine (100), the sterilization arrangement (120) comprising:
a UV-light irradiation station (122) comprising a plurality of UV-LED units (210) arranged along a feeding direction (D) of the packaging material, wherein the plurality of UV-LED units (210) are configured to be individually controllable; and
a control circuitry configured to individually adjust an UV-light irradiation intensity of the plurality of UV-LED units (210) synchronously with the passage of the specific areas (103) through the UV-light irradiation station (122) such that a relatively higher intensity is emitted when a specific area (103) is in an irradiation zone of a UV-LED unit and a relatively lower intensity is emitted when the specific area (103) is outside said irradiation zone.

2. The sterilization arrangement according to claim 1, wherein the control circuitry is configured to adjust the intensity for specific areas (103) of the packaging material selected from the group of areas consisting of: crease lines, embossed or debossed patterns, seal zones, reinforcement strips, opening device packaging material interfaces, and opening devices.

3. The sterilization arrangement according to claim 1 or 2, wherein the UV-light irradiation station (122) is a stationary station.

4. The sterilization arrangement according to any one of claims 1-3,
wherein the UV-light irradiation station (122) comprises a two dimensional matrix of UV-LED units (210),
wherein the matrix comprises a first number of columns of UV-LED units (210) arranged along the feeding direction (D) of packaging material and a second number of rows of UV-LED units (210) arranged transverse the feeding direction (D) of packaging material, and
wherein the control circuitry is configured to independently adjust the UV-light irradiation intensity of each row of UV-LED units (210) sequentially to track the passage of the repetitive specific area (103) through the UV-light irradiation station (122).

5. The sterilization arrangement according to claim 4,
wherein the UV-LED units (210) in each row of UV-LED units (210) are configured to be individually controllable,
wherein the control circuitry is configured to individually control which UV-LED units (210) in the rows of UV-LED units (210) to be adjusted based on one or both of a position and a size of the specific area (103) along a direction transverse the feeding direction (D) of the packaging material.

6. The sterilization arrangement according to any one of claims 1-5, further comprising an additional UV-light irradiation station (123) configured to subject the packaging material for UV-light irradiation along a full extension of the direction transverse the feeding direction (D) of the packaging material.

7. The sterilization arrangement according to any one of claims 1-6, wherein each UV-LED unit (210) comprise one or more individual UV-LEDs.

8. The sterilization arrangement according to any one of claims 1-7, wherein the UV-LED unit (210) are arranged to emit UV-light within the UVC spectral range.

9. A packaging machine (100) configured to fill packages (104) with a food product, the packaging machine (100) comprising:
a sterilization arrangement (120) according to any one of claims 1-8; and
a feed unit (110) configured to feed the packaging material through the UV-light irradiation station (122) of the sterilization arrangement (120).

10. The packaging machine (100) according to claim 9, wherein the packaging material is a web (102) of packaging material, wherein the feed unit (110) is configured to continuously feed the web (102) of packaging material through the UV-light irradiation station (122) of the sterilization arrangement (120).

11. The packaging machine (100) according to claim 9 or 10, wherein the control circuitry of the sterilization arrangement (120) is configured to individually adjust an UV-light irradiation intensity of the UV-LED units (210) arranged along the feeding direction (D) of packaging material based on a speed at which the feed unit (110) is feeding the packaging material through the UV-light irradiation station (122).

12. A method for sterilization of packaging material within a packaging machine (100) configured to fill packages with a food product, the packaging material comprising specific areas (103) that occur repetitively as the packaging material is fed through the packaging machine (100), the method comprising:
subjecting (S302), at a UV-light irradiation station (122) comprising a plurality of UV-LED units (210) arranged along a feeding direction (D) of packaging material, the repetitive specific areas (103) for UV-light irradiation
**characterized by** individually adjusting an UV-light irradiation intensity of the plurality of UV-LED units (210) synchronously with the passage of the specific areas (103) through the UV-light irradiation station (122) such that a relatively higher intensity is emitted when a specific area (103) is in an irradiation zone of a UV-LED unit and a relatively lower intensity is emitted when the specific area (103) is outside said irradiation zone.

13. The method according to claim 12, wherein the specific areas (103) is a type of area selected from the group of areas consisting of: crease lines, embossed or debossed patterns, seal zones, reinforcement strips, opening device packaging material interfaces, and opening devices.

14. The method according to claim 12 or 13, wherein the UV-light irradiation station (122) is a stationary station and wherein the packaging material is a web (102) of packaging material, the method further comprises continuously feeding (S201) the web (102) of packaging material comprising the specific areas (103) through the UV-light irradiation station (122).

15. The method according to any one of claims 12-14, wherein individually adjusting the UV-light irradiation intensity of the plurality of UV-LED units (210) as a specific area (103) passes through the UV-light irradiation station (122) is performed based on a feeding speed of the packaging material through the UV-light irradiation station (122).
